# EUROPEAN PATENT SPECIFICATION

(11) **EP 2 308 435 B1**
(45) Date of publication and mention of the grant of the patent: **08.01.2014**
(21) Application number: 09802410.2
(22) Date of filing: 27.07.2009
(51) Int. Cl.: A61F 13/42, G08B 21/20

(54) **DISPOSABLE DIAPER STATE DETECTING DEVICE AND DISPOSABLE IDAPER STATE MONITORING SYSTEM**
EINWEGWINDEL-STATUSERKENNUNGSVORRICHTUNG UND EINWEGWINDEL-STATUSÜBERWACHUNGSSYSTEM
DISPOSITIF DE DÉTECTION D'ÉTAT DE COUCHE JETABLE ET SYSTÈME DE SURVEILLANCE D'ÉTAT DE COUCHE JETABLE

(30) Priority: 28.07.2008 CN 200810142520; 28.07.2008 CN 200810142519; 08.08.2008 CN 200810142331; 18.08.2008 CN 200810142385; 01.02.2009 CN 200920129698 U
(43) Date of publication of application: 13.04.2011
(73) Proprietor: Ckicom Technology Limited, Hong Kong (HK)
(72) Inventor: XU, Fei, Hong Kong (CN); HUANG, Geng, HHong Kong (CN)
(74) Representative: Hocking, Adrian Niall
(86) International application number: PCT/CN2009/072938
(87) International publication number: WO 2010/012217

(56) References cited:
- WO-A2-97/42613
- CN-A- 1 226 855
- CN-A- 1 489 983
- CN-A- 1 645 425
- CN-A- 101 166 495
- CN-U- 2 076 835
- CN-U- 2 076 835
- CN-Y- 2 362 471
- CN-Y- 2 768 733
- CN-Y- 200 957 137
- JP-A- 2005 000 602
- US-A1- 2006 244 614

## Description

### CROSS-REFERENCE TO RELATED APPLICATIONS

This application claims priorities of Chinese patent application No. 200810142520.0 filed on July 28, 2008, Chinese patent application No. 200810142519.8 filed on July 28, 2008, Chinese patent application No. 200810142331.3 filed on August 8, 2008, Chinese patent application No. 200810142385.x filed on August 18, 2008, and Chinese patent application No. 200920129698.1 filed on February 1, 2009, the entire contents of which are hereby incorporated by reference.

The present application relates to a disposable diaper condition detecting device and monitoring system, and particularly to a wireless network device and system to realize wireless urine-wetness induction and urine-wetness condition indication suitable for disposable diapers.

### BACKGROUND

Disposable diapers (or paper diapers) are hygiene goods. They are very popular at home, in hospitals and at care centers. Disposable diapers are discarded after use and no doubt are more hygienic and convenient relative to conventional diapers. A disposable diaper is usually formed of a dry layer (inner layer), an absorbing layer (interlayer) and an anti-leakage layer (outer layer). The dry layer is the innermost layer of the diaper. When a user urinated inside the diaper, urine can pass through the dry layer and can be absorbed by the absorbing layer so that the dry layer can remain dry. However, the dryness is relative. In one aspect, the absorbing capacity of the absorbing layer is limited. In another aspect, the urine in the absorbing layer can also disperse moist air and the anti-leakage layer is not air permeable enough because it has to prevent leakage of urine. The gas generated by the decomposition of urine inside the absorbing layer may remain in the diaper for a long time causing irritation of the skin of the user. Therefore, the diaper should be changed after it is wetted to keep the user's skin healthy.

Since a disposable diaper is relatively tight, it is difficult in normal situation to observe whether the diaper has been wetted. Particularly when the caretakers are rather busy, it is easy to neglect the changing of the wetted diaper that causes skin problem of the user. Hence, there is a need in practical life an effective indicating method of reminding caretakers to change the diapers so that the health of the user's skin can be protected. Disposable diapers are suitable for both adults and babies. It is indispensable in particular for patients and elders who suffer from incontinence. Hence, disposable diapers are largely used in organizations such as hospitals, sanatoriums, nursing homes and care centers, etc. In order to raise the quality of service and the level of caretaking in those organizations, and to reduce the problem created by shortage of man power, there is really a need to establish an effective, network-oriented urine-wetness induction and urine-wetness condition indicating/alarming system.

However, the existing stand-alone electronic urine-wetness acousto-optic alarm devices cannot satisfy the need. The reason is that in most situations the diaper is worn inside. Acousto-optic alarm can easily be neglected in a relatively noisy and busy environment. Also, if the alarm is too loud, it can easily disturb other patients. In reality, this normally does not work.

The above description of the background is provided to aid in understanding a disposable diaper condition detecting device and monitoring system, but is not admitted to describe or constitute pertinent prior art to the disposable diaper condition detecting device and monitoring system disclosed in the present application, or consider any cited documents as material to the patentability of the claims of the present application.

### SUMMARY

The technical problem to be solved in the present application is to aim at the technical problem of the existing product and provide a disposable diaper condition detecting device and monitoring system. Particularly, it provides a wireless network device and system to realize wireless urine-wetness induction and urine-wetness condition indication suitable for disposable diapers. The system can effectively remind caretakers to carry out the necessary caring tasks without disturbing other people. It is convenient to use, reliable, clean and hygienic, and relatively low in cost of using the device.

In order to solve the above-mentioned technical problem, the present application provides a disposable diaper condition detecting device. The disposable diaper condition detecting device includes a disposable diaper, conductive induction wires and an alarming device, the disposable diaper including an outer layer, an interlayer and an inner layer, the induction device being disposed on the outer layer, or between the outer layer and the inner layer of the disposable diaper for detecting urine-wetness condition of the disposable diaper and generating a urine-wetness condition induction signal, the alarming device being integrally formed with the conductive induction wires, or combinable with or separable from the conductive induction wires, and being electrically coupled to the conductive induction wires when combined together for producing an alarm according to the urine-wetness condition induction signal generated by the conductive induction wires, characterized by sampling means for activating the conductive induction wires to sample for the presence of urine, the sampling means including a battery-chargeable capacitor attached at the input end of the conductive induction wires, a sample input loop being triggered when a charge of the capacitor reaches a predetermined level, wetness monitoring means for determining wetness and degree of wetness, whereby a processor determines the wetness and the degree of wetness of the diaper (10, 50) according to the time triggered by the charging of the capacitor.

The present application further provides a disposable diaper condition monitoring system. The disposable diaper condition monitoring system including a disposable diaper condition detecting device in accordance with the first aspect of the invention and a disposable diaper condition displaying device, the disposable diaper condition displaying device being adapted to display diaper condition signals according to urine-wetness condition signals or urine-wetness alarm signals transmitted from the detecting device, the disposable diaper condition detecting device further comprises a wireless sensor, the wireless sensor comprising a sensing input device, transmittable sensor ID, and a wireless transmitting device, the transmittable sensor ID is transmitted via the wireless transmitting device when the sensor enters a triggered condition; and the disposable diaper condition displaying device comprises a wireless display, the wireless display comprising a wireless receiving device, a data processing device, and a displaying device, whereby after the display receives the transmittable sensor ID transmitted from the sensor, the data processing device determines its relationship with the display and, if related, displays on the displaying device the signal related to the sensor.

The disposable diaper condition detecting device disclosed in the present application has the following advantages:
Firstly, it is an electronic-enabled detecting device which can carry out active urine-wetness alarm.
Secondly, the sensor is far away from the skin in use so that it can prevent erroneous activation caused by sweating or light excretion.
Thirdly, the sensor connected to the interlayer of the diaper is disposable. The sensor is discarded together with the diaper after use. This ensures a clean and hygienic usage.

Finally, the alarm and the sensor are separable. The alarm can be used repeatedly. This effectively reduces the cost of using the device.

The disposable diaper condition monitoring system of the present application has the benefits in that:
(i) It is a wireless induction network system. The position of the sensors does not affect the alarm effect. This can effectively solve the problem that the normal acousto-optic alarm system is not suitable for hospitals and care centers.
(ii) It is easy to establish a networking and centralized wireless system suitable for use in organized and sized application.
(iii) The sensor is provided with call function to solve special needs of different patients, or to be used in emergency situations.
(iv) Voice intercom function is provided on the wireless display in the patient's room and the data terminal at the caring center. This is a direct and effective way of communication. This is an important function of the network system.
(v) The data terminal can further be connected to a computer for processing all the data of the sensors in the system. This can realize the recording of the condition of elders/patients and raise the quality of caring and level of service of the nursing organizations.

Although the disposable diaper condition detecting device and monitoring system disclosed in the present application are shown and described with respect to certain embodiments, it is obvious that equivalents and modifications will occur to others skilled in the art upon the reading and understanding of the specification. The present application includes all such equivalents and modifications, and is limited only by the scope of the claims.

### BRIEF DESCRIPTION OF THE DRAWINGS

Specific embodiments of the disposable diaper condition detecting device and monitoring system disclosed in the present application will now be described by way of example with reference to the accompanying drawings wherein:
FIG. 1 is an illustrative diagram of the network of a disposable diaper condition monitoring system of the present application;
FIG. 2 is an illustrative diagram of an embodiment of a urine-wetness induction alarm in an embodiment of a wireless sensor in accordance with a first aspect of the present application;
FIG. 3 is a side view of the urine-wetness induction alarm in the wireless sensor of Figure 2(before assembly);
FIG. 4 is a side view of the urine-wetness induction alarm in the wireless sensor of Figure 2 (after assembly);
FIG. 5 shows the front and rear views of the urine-wetness induction alarm in the wireless sensor of Figure 2;
FIG. 6 shows the side and cross sectional side views of a sensing input device and a separable urine-wetness alarm in yet another embodiment of the wireless sensor in accordance with the first aspect of the present application;
FIG. 7 shows the front view of the sensing input device and the rear and front view of the separable urine-wetness alarm of Figure 6;
FIG. 8 shows the fragmentary cross sectional view of the electronic wetness-sensing diaper for use in cooperation with the wireless sensor of Figure 6;
FIG. 9 is a circuit diagram of the urine-wetness induction alarm in the wireless sensor of Figure 2 or 6, in accordance with the first aspect of the present application;
FIG. 10 is a block diagram of a wireless sensing alarm circuit in the wireless sensor of Figure 2 or 6, in accordance with the second aspect of the present application;
FIG. 11 is a block diagram of the wireless display circuit in the wireless sensor of Figure 2 or 6, in accordance with the second aspect of the present application;
FIG. 12 is a block diagram of a remote data terminal circuit in a remote data terminal, in accordance with the second aspect of the present application;
FIG. 13 is an illustrative diagram (cross sectional side view) of the structure/assembly of the electronic wetness-sensing diaper and the urine-wetness alarm in yet another embodiment of the wireless sensor, in accordance with the first aspect of the present application;
FIG. 14 is an illustrative diagram (front view) of the structure/assembly of the electronic wetness-sensing diaper and the urine-wetness alarm in the wireless sensor of Figure 13;
FIG. 15 is an illustrative diagram of conductive induction wires screen printed on the inner edge of the outer layer of the diaper, in accordance with the first and second aspects of the present application; and
FIG. 16 is a block diagram of the wireless urine-wetness alarm system with color condition indication, in accordance with the second aspect of the present application.

### DETAILED DESCRIPTION

The disposable diaper condition detecting device and monitoring system of the present application will be further described with reference to the accompany drawings.

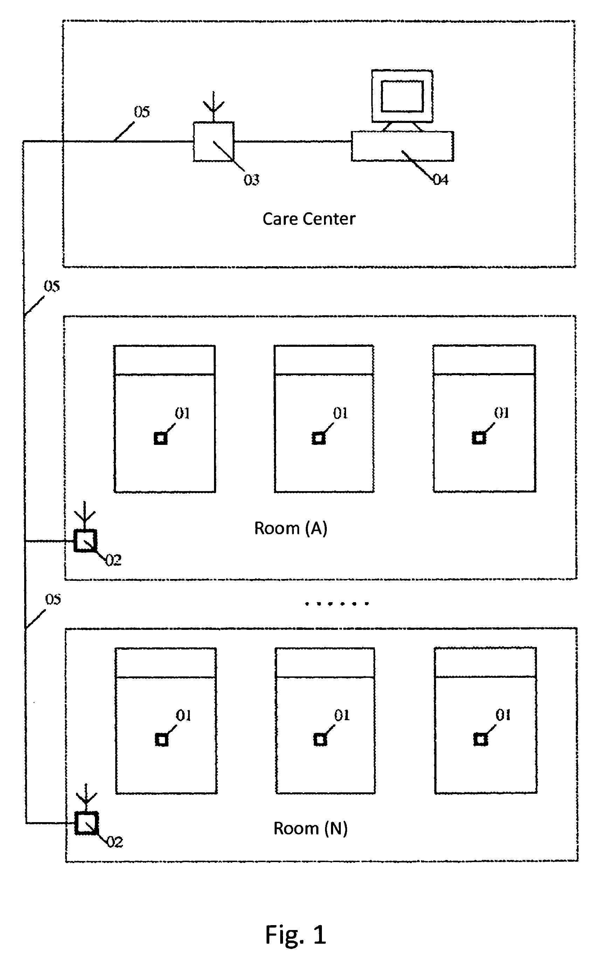

FIG. 1 is an illustrative diagram of the network of an embodiment of a disposable diaper condition monitoring system of the present application. Generally speaking, a nursing system includes at least one nursing center and a number of patient's rooms (rooms). Usually there are one or more nursing beds (patient's bed) in one room. In the present embodiment, there are N rooms and three beds in each room. In the present embodiment, each bed may be equipped with a wireless sensor 01, and each room may be equipped with a wireless display 02. The nursing center may be equipped with a remote data terminal 03. Radio frequency may be employed to connect between the wireless sensors 01 and the wireless display 02. Radio frequency (with antenna) may also be employed to connect between the wireless display 02 and the remote data terminal 03, or wired connection or electric carrier wave 05 may be employed to carry out the connection. The remote data terminal 03 may be used independently for data receiving and displaying. The remote data terminal 03 may further be connected to a computer 04 to realize networking and computerizing the real time data display and recordation.

It can be seen from the illustrative diagram that the system of the present application has two main components which are the wireless sensors 01and the wireless display 02, and may further include the remote data terminal 03. Each component will be analyzed in detail with reference to the accompany drawings so as to form a complete wireless sensing and condition displaying system.

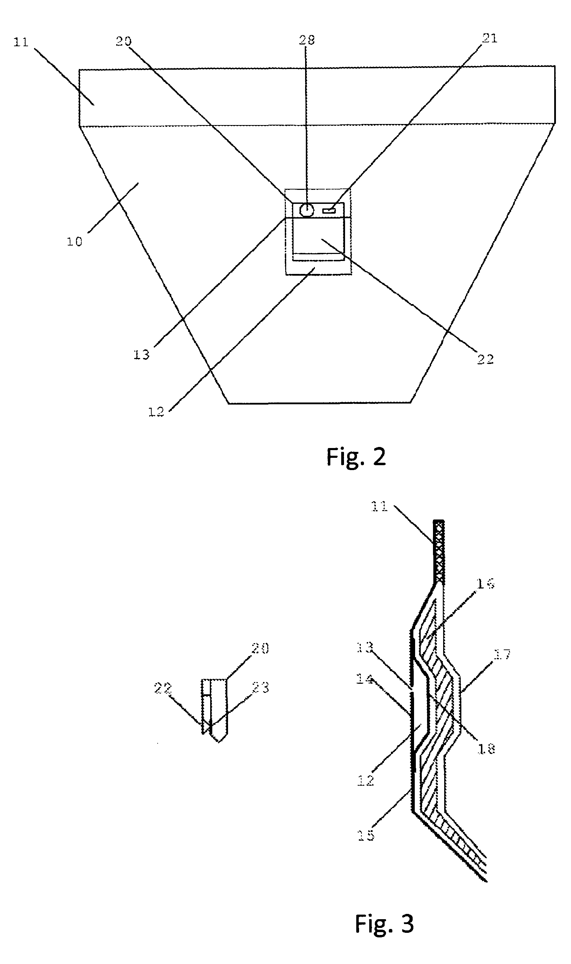

FIG. 2 is an illustrative diagram of the application of the disposable diaper condition detecting system of an embodiment of the wireless sensor 01 in accordance with a first aspect of the present application.

It is necessary to indicate that the present application provides a disposable diaper condition detecting system, including a disposable diaper, conductive induction wires and an alarming device;
the disposable diaper comprising an outer layer, an interlayer and an inner layer;
the conductive induction wires being disposed on the outer layer, or between the outer and inner layers, for detecting urine-wetness condition of the disposable diaper and generating a urine-wetness condition induction signal;
the alarming device being integrally formed with the conductive induction wires, or combinable with or separable from the conductive induction wires, and
the alarming device being electrically coupled to the conductive induction wires when combined together for sounding alarm according to the urine-wetness condition induction signal generated by the conductive induction wires, characterized by
sampling means for activating the conductive induction wires to sample for the presence of urine, the sampling means including a battery-chargeable capacitor attached at the input end of the conductive induction wires, a sample input loop being triggered when a charge of the capacitor reaches a predetermined level, wetness monitoring means for determining wetness and degree of wetness, whereby a processor determines the wetness and the degree of wetness of the diaper according to the time triggered by the charging of the capacitor.
Since the disposable diaper of the present application is different from the disposable diaper of the existing technology, the disposable diaper in the following embodiments will be termed electronic sensing diaper or electronic wetness-sensing diaper in order to facilitate the description of the diaper and to emphasize that the diaper of the present application has the function of electronically sensing urine-wetness.
In the present embodiment, the alarming device can be a urine-wetness alarm. The urine-wetness alarm and the conductive induction wire can be combined together to form a wireless sensor for transmitting diaper condition signals or urine-wetness alarm signals.
A main application is to provide a wireless disposable diaper condition monitoring system for hospital, sanatorium and nursing center. It is also called a wireless urine-wetness induction and alarm system. Hence, a disposable diaper condition detecting device, and a wireless urine-wetness induction and alarming device of the system are introduced in the present application. This is an embodiment disclosed in the application. Although not within the scope of the present invention, the system may also be suitable for other wireless condition detection, and can be achieved by merely equipping the system with the necessary sensing input device. For example, it can be applied in detecting intravenous drip (fluid infusion) in hospital, or the position of patients. All it requires is a medical fluid sensor or a position sensor. This can prevent the backward blood flow occurred after the completion of the transfer of medical fluid of the intravenous drip, and can prevent patients from leaving the patient's area on their own so as to ensure the health and safety of patients.
In FIG. 2, reference numeral 10 denotes the disposable diaper adopted (specially used) in the present application. The shape of the diaper and the way of wearing the diaper are the same as a traditional diaper. The difference is that a stand-alone dry space 12 can be provided at a certain region (in the figure, the front or back of the diaper) outside the diaper 10. An opening (entrance) 13 can be provided at the dry space 12. An integrated alarm 20 may be inserted through the entrance. An indicating light (light-emitting diode, LED) 21 may be provided on the alarm 20 for indicating the operating condition or the urine-wetness condition. A call button 28 may be provided adjacent to the indicating light 21 for the user of the diaper to call the caretakers on duty when necessary. A flexible clip 22 may further be provided on the alarm 20. The flexible clip 22 can be clipped onto the outer edge of the stand-alone dry space 12 in order to secure the alarm 20 on the diaper 10.
The main concept in FIG. 2 is to solve the technical problem in existing urine-wetness induction and acousto-optic alarm system. Firstly, it is a wireless urine-wetness induction alarming device. The position of the sensors does not affect the alarm effect. This can effectively solve the problem of the conventional acousto-optic alarms that are not suitable for hospital and nursing center. Secondly, it can facilitate the establishment of a centralized monitoring and caring system, which is particularly suitable in organized and sized monitoring and caring applications. Thirdly, one end of the wireless sensor is provided with call function to solve special needs of different patients, or to be used in emergency situations. Fourthly, voice intercom function is provided on the wireless display in the patient's room and the data terminal at the caring center. This is a direct and effective way of communication.

FIG. 3 is a side view of the first embodiment of the urine-wetness induction alarm of the wireless sensor 01 in accordance with the first aspect of the present application (before assembly). In the figure, reference number 11 represents the waistline of the diaper, reference numeral 15 represents an outer layer (anti-leakage layer) of the diaper, reference numeral 16 represents an absorbing layer (interlayer) of the diaper, and reference numeral 17 represents an inner layer (dry layer) of the diaper. A slit opening 13 may be provided on the outer layer 15 of the diaper. An anti-seepage protective layer 18 can be provided underneath the slit opening 13. The protective layer 18 covers underneath the slit opening 13 of the diaper. The four sides of the protective layer 18 can be adhered on the inner surface of the outer layer of the diaper. The protective layer 18 can prevent the leakage of urine at the slit opening 13. The protective layer 18 and the outer layer of the diaper together form a stand-alone dry (leak free) space 12. This dry space 12 can be reached directly from the outside of the diaper through the slit opening 13, and can be used externally by a user. In the present application, the dry space 12 is mainly used for the accommodation (placement) of the urine-wetness induction alarming device. Conductive induction wires leading to the absorbing layer of the diaper may be included in the dry space 12 so as to electrically couple the absorbing layer of the diaper to the urine-wetness induction alarming device. The reference numeral 20 denotes an integrated (relative to the wireless sensor in the second embodiment) urine-wetness induction alarm (or the main body of the urine-wetness alarm containing a urine-wetness induction alarm circuit), reference numeral 22 denotes a flexible clip 22, and reference numeral 23 denotes contact electrodes. Details will be described in the following examples and figures.

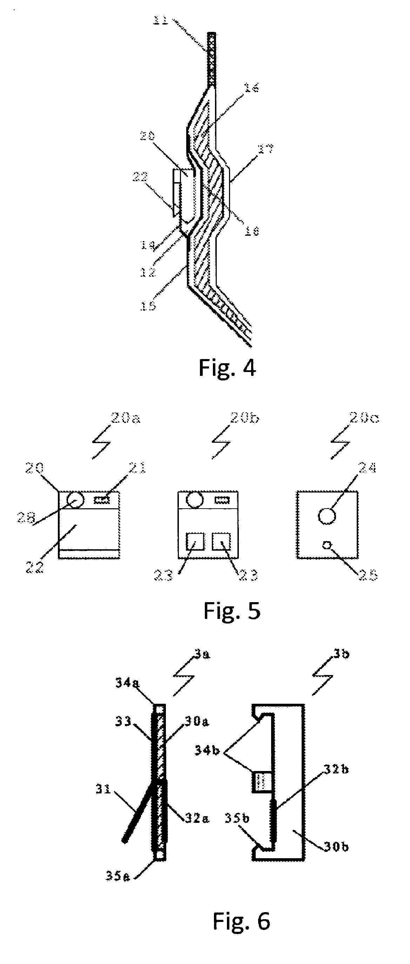

FIG. 4 is a side view of the first embodiment of the urine-wetness induction alarm of the wireless sensor in accordance with the first aspect of the present application and a cross sectional side view of the diaper (after assembly). The reference numerals used in FIG. 4 are the same as those used in FIG. 3. The difference is that the urine-wetness induction alarm 20 is placed inside the dry space 12 of the diaper. The flexible clip 22 of the urine-wetness induction alarm clips onto the outer edge 14 (a portion of the outer layer 15 of the diaper) of the dry space 12 from outside the diaper. In one aspect, the flexible clip 22 can be used to hold the urine-wetness induction alarm onto the diaper. In another aspect, it can provide a reliable contact (enhance the resilient force at the contact) between the conductive induction wires (in contact with the absorbing layer of the diaper) adhering to the inner edge of the outer layer of the diaper and the contact electrodes on the urine-wetness alarm.

FIG. 5 shows the front view (20a) and the rear view (20c) of the urine-wetness induction alarm of the wireless sensor 01 of Figure 2. The urine-wetness induction alarm may include a main body 20 of the urine-wetness alarm, which may include an alarm circuit, a LED condition indicator 21, a resilient clip 22 and a call button 28. Reference number 20b denotes the front view of the alarm without the resilient clip 22. As shown in the figure, there may be two contact electrodes 23. Since the contact electrodes 23 are located underneath the resilient clip 22, these electrodes may not be visible in the front view 20a of the urine-wetness alarm. The reference numeral 24 in the rear view 20c represents a button. The button 24 may be employed to switch the power of the urine-wetness alarm on or off, or may have a testing capacity, or may be used for the synchronization of the sensor ID mentioned below. A user can use different periods of the time for which the button is pressed to distinguish different functions. For example, a long pressing on the button may be used for on/off switching, a short pressing may be used for testing, and pressing the button two times or three times may be used for generating the sensor's random ID and synchronizing with the wireless display. In the rear view 20c, there may also be a reset button 25 for resetting the circuit of the urine-wetness induction alarm.

FIG. 6 shows a sensing input device and a detachable urine-wetness alarm in yet another embodiment of the wireless sensor in accordance with the first aspect of the present application. The difference between the first and second embodiments is that the sensing input device and the urine-wetness alarm are integrally formed. It does not have a stand-alone sensing input device because the sensing input device is contained inside the urine-wetness alarm. However, in the second embodiment, the sensing input device and the urine-wetness alarm can be separated. The sensing input device can be disposable. In use, the sensing input device can be adhered onto the disposable diaper and can be disposed of together with the disposable diaper. The detachable urine-wetness alarm can be used repeatedly for many times because it can be detachable. The diaper in the first embodiment is different from the diaper in the second embodiment. The diaper in the first embodiment is a diaper for special use. It has an internal conductive induction wire and a stand-alone dry space for accommodating the urine-wetness induction alarm. However, in the second embodiment, the diaper is an ordinary disposable diaper. Since an ordinary diaper is not provided with an internal conductive induction wire, it requires an external sensing input device with a sensing electrode that can be inserted into the diaper through a slit opening formed on the outer layer of the diaper, and can be in contact with the absorbing layer of the diaper. Therefore, it will inevitably be contaminated in use and needs to be discarded together with the diaper.

The reference numeral 3a in FIG. 6 denotes the sensing input device. The sensing input device may include a pair of sensing electrodes 31. The sensing electrodes 31 can be fixed by an insulating gasket 30a, pass through the insulating gasket 30a and reach the other side of the gasket. The portions of the sensing electrodes 31 at the other side of the gasket represented by reference numeral 32a can be attached to the insulating gasket 30a. The insulating gasket 30a may further include a non-dry adhesive layer 33 and positioning notches 34a and 35a. The reference numeral 3b in FIG. 6 denotes the separable urine-wetness alarm. The urine-wetness alarm may include a main body 30b containing therein an alarm circuit. The reference numeral 32b represents contact electrodes of the urine-wetness alarm. The reference numerals 34b and 35b represent positioning catches of the urine-wetness alarm. The operating procedures of the urine-wetness alarm are described below. Firstly, a slit opening or slit string can be formed on the portion (e.g. the front) of the diaper that requires urine-wetness detection. The slit string may include a pulling handle for pulling open a slit to form a slit opening on the outer layer of the diaper. Secondly, the sensing electrodes 31 can be inserted into the slit opening so that they are in contact with the absorbing layer of the diaper. Thirdly, the insulating gasket is attached to the diaper by means of the non-dry adhesive layer. In one aspect, it can have a fixing function. In another aspect, it can prevent leakage of urine through the slit opening of the diaper. Fourthly, the main body 30b of the alarm can be fastened onto the insulating gasket 30a by means of the catches 34b and 35b. This completes the fixing and assembling of the sensing input device and the urine-wetness alarm. In the assembled condition, the contact electrodes 32b can be in contact with the sensing electrodes 32a so that to realize electrical coupling between the absorbing layer of the diaper and the alarm.

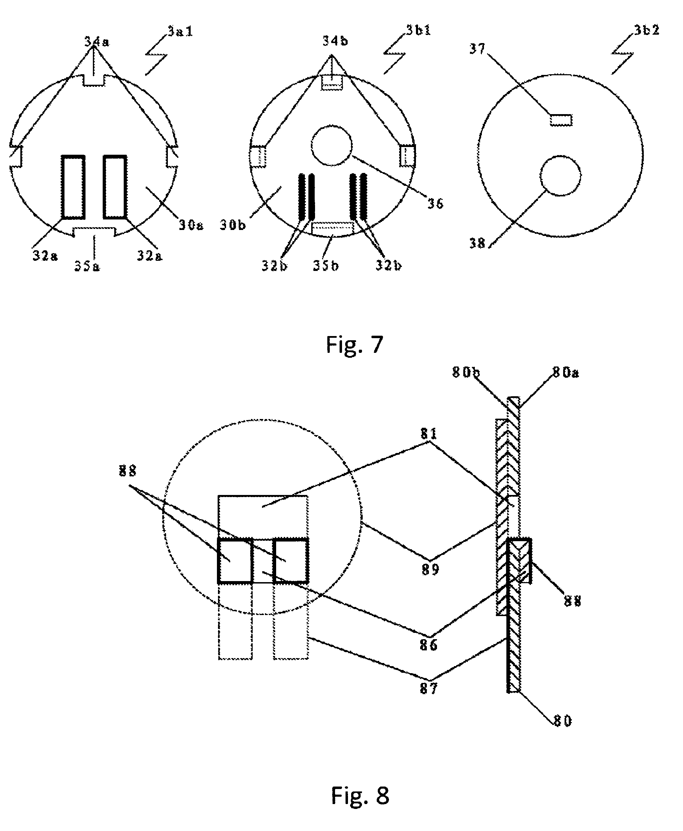

FIG. 7 shows the front view (3a1) of the sensing input device, and the rear and front views (3b1 and 3b2) of the urine-wetness alarm of Figure 6. The positioning notches 34a, 35a at the outer edge of the insulating gasket and the positioning catches 34b, 35b on the alarm are clearly illustrated in FIG. 7. The dimensions of the positioning notches 34a, 35a and the positioning reverted catches 34b, 35b are different (the dimensions of positioning notch 35a and the positioning catch 35b are bigger). This difference in structure can prevent the possibility of incorrect coupling between the sensing input device and the urine-wetness alarm. The sensing electrodes 32a associate with the contact electrodes 32b. When the sensing input device and the urine-wetness alarm are in an assembled condition, the sensing electrodes 32a can be electrically coupled to the contact electrodes 32b. In the rear view (3b1) of the urine-wetness alarm, it can be seen that there is a button 36 for switching the power on or off, or for wireless testing, or may be used for sensor ID synchronization. A user can use different periods of the time for which the button is pressed to distinguish different functions. For example, a long pressing of the button may be adopted for on/off switching, a short pressing for testing, and pressing the button two times or three times for generating the sensor's random ID and synchronizing with the wireless display. In the front view (3b2) of the urine-wetness alarm, there may also be an LED condition indicator 37 and a call button 38 for condition indication and the realization of call function.

The sensing input device in the second embodiment of the wireless sensor 01 described above senses urine wetness through the diaper's slit opening that allows the contact of the sensing electrodes with the absorbing layer of the diaper. However, a disadvantage of this method is that its sensing area is relatively narrow. It may not be able to sense the wetness if it is located at a region farther away from the slit opening of the diaper. A solution is the addition of two conductive induction wires at the center of the diaper. FIG. 8 is an illustrative diagram of the slit portion of the electronic diaper that matches the sensing input device of the wireless sensor of Figure 6. In the figure, reference numeral 80 denotes the outer layer (anti-leakage layer, longitudinal cross section) of the diaper, reference numeral 80a denotes the outer edge of the outer layer of the diaper, reference numeral 80b denotes the inner edge of the outer layer of the diaper, and reference numeral 81 denotes a slit opening or slit line provided on the outer layer of the diaper. In case of a slit string, it may be provided with a pulling handle for pulling open the slit line to form a slit opening on the outer layer of the diaper. The slit opening on the outer layer of the diaper may have an inverted U-shaped structure. The outer layer material at the slit opening can be retained and flipped over to the outer edge of the outer layer (called flipped side 86 for short). The inner edge of the outer layer of the diaper may be further provided with conductive induction wires 87. The conductive induction wires 87 can pass the flipped side 86 through the slit opening and can expose at the outer edge 88 of the outer layer of the diaper. This can facilitate the installation of the urine-wetness sensor at the slit opening and the realization of electrical connection between the sensing electrodes of the urine-wetness sensor and the conductive conduction wires. It can be seen in the figure that there is a protective layer 89 for the prevention of leakage of urine at the slit opening. The conductive induction wires 87 in the embodiment can be in contact with the above-mentioned sensing electrodes, and can carry out detection of urine-wetness condition on a larger area of the diaper. The conductive induction wires 87 in the embodiment may be internally formed during the manufacturing of the diapers, and can be adhered or printed on the inner edge of the outer layer of the diaper. The material of the conductive induction wires 87 may be any soft conductive media such as conductive filaments, conductive paper, conductive fabric, and conductive fiber, etc. The conductive induction wires 87 may be printed directly on the inner edge of the outer layer of the diaper using conductive ink. Since the conductive induction wires 87 can be distributed in different regions on the diaper, the sensing area can be broader than that in the first embodiment. According to the illustrated embodiment, there are two conductive induction wires. In practice, there can be more conductive induction wires to increase the sensing area or to designate certain special sensing areas.

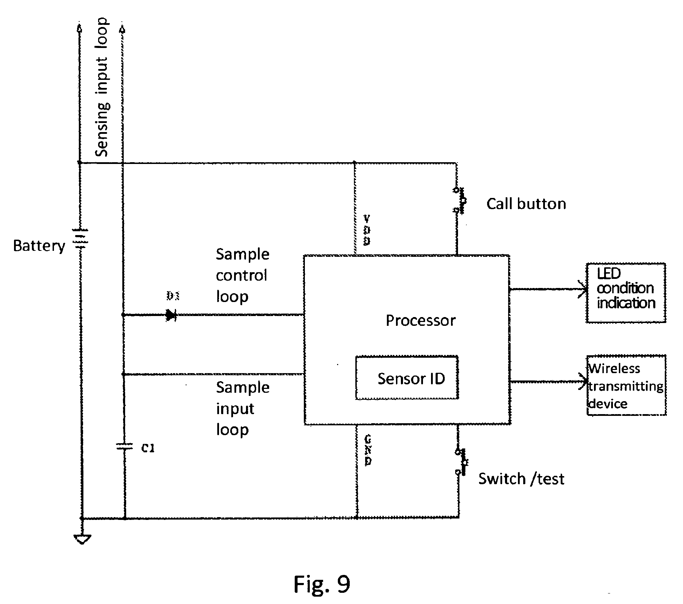

FIG. 9 is a circuit diagram of an embodiment of the urine wetness induction alarm of the wireless sensor 01, in accordance with the first aspect of the present application. The core of the circuit is a processor, or a device containing a processor, such as a single chip microprocessor or a special digital integrated circuit, etc. The figure shows the adoption of a capacitance-resistance type sensing input loop. The loop can detect whether urine wetness has occurred. The loop can even detect the degree of urine wetness because it can generate a quantitative input. The key of this circuit is the attachment of a capacitor C1 at the input end of the conductive induction wires. The capacitor may be connected in series in the loop of the conductive induction wires. A battery can charge the capacitor through the resistance in the conductive induction wires. When charging reaches a certain voltage level, a sample input loop can be triggered. According to the time triggered by the charging, the processor can determine whether the diaper is wetted by urine and the degree of wetness, etc. It is because the time and the resistance between the conductive induction wires are related (inversely proportional relationship). Further, the resistance between the conductive induction wires and the degree of urine wetness are related. There may also be a sample control loop. This loop can be connected to and controlled by the processor. When urine-wetness induction (sampling) is carried out, the processor sends out a low voltage level via the sample control loop rendering the charging of the capacitor C1 via diode D1. At this moment, the sample input loop is at a low voltage level. Then, the processor sends out a high voltage level via the sample control loop rendering the termination of charging (reversely cutoff the diode in series with the control loop). At this moment, the battery can charge the capacitor C1 via the above-mentioned loop until the sample input loop becomes high voltage and triggers the processor. If the diaper is in a dry condition, the resistance between the conductive induction wires is at high impedance and the charging rate of the capacitor C1 is very slow. It takes a relatively long period of time for the sample input loop to change from a low voltage level to a high voltage level. When the diaper is in a wet condition, the resistance between the conductive induction wires is at low impedance and the charging rate of the capacitor C1 is relatively fast. It takes a relatively short period of time for the sample input loop to change from a low voltage level to a high voltage level. At this time, it can be confirmed that the diaper is at a wet condition. Furthermore, the shorter the period of time, the more serious is the urine-wetness condition. The use of the processor renders the realization of the quantitative signal input, and makes urine wetness detection more accurate and reliable. The device may further include a LED condition indicator for the indication of operating condition and alarm condition. For example, a flashing green light may indicate that the diaper is at in dry condition. A flashing red light may indicate that the diaper is in a wet condition. A flashing orange light (red and green lights flashing at the same time) may indicate a low voltage condition, etc. These conditions can be realized by using a two-color LED.

The circuit in FIG. 9 may further include a wireless transmission device which can transmit wireless alarm signal. A cooperative wireless display 02 can receive a wireless alarm signal and generate a corresponding display output. Since the system may include more than one wireless sensor 01, it is necessary for the wireless display 02 to effectively recognize the wireless sensor 01 that sends out the wireless signal. The wireless signal of the sensor must include the sensor's address or identity code (sensor ID). According to the present embodiment, such ID may be provided in the processor. The processor may transmit the alarm signal with the sensor ID to the wireless transmission device, which realizes digitalized signal transmission. The device may further include an on/off button or test button. The button can be used to switch the alarming device on or off. The button can also be used to test the transmission of the alarm. Furthermore, this button can be used to realize the synchronization with the ID of the wireless display 02.

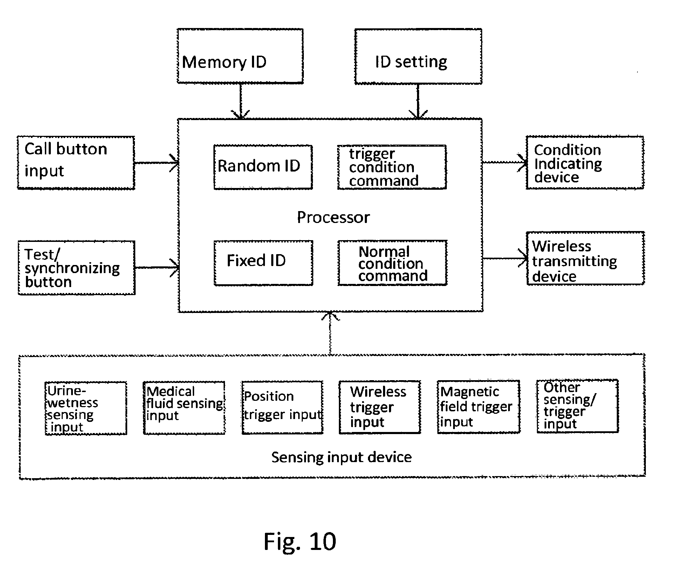

FIG. 10 is a block diagram of the wireless sensing alarm circuit of the wireless sensor 01, in accordance with the second aspect of the present application. It may include a processor and various units/devices connected to the processor such as sensing input device, sensor ID unit, wireless transmission device, etc. When the sensing input device is triggered, the processor can transmit sensor ID through the wireless transmission device. The sensor ID unit may include ID setting device (e.g. toggle switch), and storage ID (stored in a storage that cannot be lost easily) outside the processor. The ID inside the processor may include fixed ID and random ID. Fixed ID means the ID that is preset inside the processor before the product is delivered out of the factory. Random ID means the ID that is generated from the processor through certain method or calculation during use. For example, it can be generated at the first power-up of the wireless sensor (e.g. make use of the combination of internally stored random numbers during power-up). It can also be generated during synchronization of the wireless display 02 and the wireless sensor 01 via a synchronizing button. In the present system, the value of the ID, whether large or small, is not important. It is important that each ID of the sensor is different so that it can distinguish different sensors. It is assumed that the sensor can adopt the pressing of the synchronizing button twice consecutively in order to synchronize with the wireless display 02, and transmit to the wireless display 02 the time interval of the two consecutive pressings of the button. It is further assumed that the time interval of the two consecutive pressings of the button is 0 to 1 second, and the precision of the counter is 1 million time per second. Then, the probability of repeating a sensor ID generated based on the above assumptions is 1 out of one million. As far as the present system is concerned, this is acceptable and is rather close to an ideal situation.

The sensor input device may include urine-wetness sensing input, medical solution sensing input, position trigger input, wireless trigger input, magnetic field trigger input, and other sensor input. Wireless trigger input and magnetic field trigger input are position trigger inputs. When entering the wireless trigger or magnetic field trigger region (for example, install the trigger transmitting device at a designated exit or entrance), the wireless sensor can be triggered and transmits its ID code such that the monitoring system can know the position of the wireless sensor 01. This can realize the chasing of the position of the wireless sensor 01.

The system may further include a call button input and a test/synchronization button input. The call button input can be used for calling purposes. When the call button is pressed, the wireless sensor 01 can transmit a call signal with a sensor ID, and can display on the wireless display 02 a signal relating to the sensor. At the same time, the call signal can further transmitted in the network system to the remote data terminal 03 at a care center for display. The system may further include a condition indicator for indicating the working condition of the wireless sensor 01, or for indicating the alarm condition. The trigger condition command and the normal condition command inside the processor can be the designated data package transmitted together with the sensor ID. This makes the wireless display 02 not only know the sensor ID of the transmitting signal, but also know the current condition and renew the condition based on its changes. For example, when the system has a urine-wetness situation, the wireless sensor 01 transmits a urine-wetness trigger alarm commend that makes the red indicating light of the wireless display 02 that received the corresponding signal flash. When the diaper is changed and is in a dry condition, the wireless sensor 01 transmits a normal condition command that makes the flashing red indicating light of the wireless display 02 stop flashing, and turns the green indicating light on in order to show that the system has returned to a normal condition.

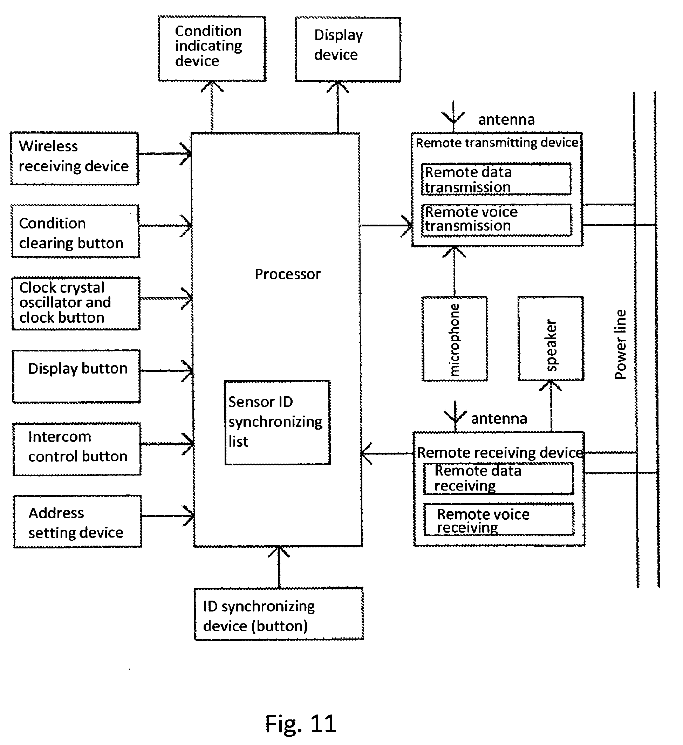

FIG. 11 is a block diagram of the wireless display circuit of the wireless display 02, in accordance with the second aspect of the present application. Same as the wireless sensor, the core of the wireless display is a processor. The first device connected to the processor is a wireless receiving device. The wireless receiving device can be used to receive wireless signals transmitted from the wireless sensor, and analyze the received signals. If the sensor ID included in the signal corresponds to the ID in the sensor ID synchronization list stored in the wireless display 02, the corresponding sensor signal can be displayed by the display device connected to the processor so as to realize wireless transmission and remote display of monitor/alarm signals. There may be an ID synchronizing device (ID synchronizing button) connected to the processor. ID synchronization is an important step of establishing relationship between the wireless sensor 01 and the wireless display 02. The wireless display 02 has to first carry out ID synchronization with the wireless sensor 01, before it can recognize the corresponding wireless sensor 01. In the illustrated embodiment, the sensor ID may adopt random ID (this can avoid the problem when manufacturing the set ID and the necessity of an external ID memory and an ID setting device). However, the wireless display 02 does not recognize (does not know) the random ID, and does not know what to display when the ID is received. Therefore, it has to carry out ID synchronization first.

FIG. 11 also shows a clock crystal oscillator and a clock button module. The wireless display 02 of the present embodiment may further have the function of a digital clock. The clock crystal oscillator is the signal source of time. The processor can be the counter of time to drive the time display. A time button may be used to input the initial time and to adjust the time when necessary. The display device can be used to output the time display (display the hour/minute). In the present embodiment, the display device is used to display the time when it is in the clock mode. Further, the display device can be used to display the sensor's signal (the room number and bed number where the sensor is located) when it is in the sensor synchronization and urine-wetness alarm mode.

The wireless display 02 in the present embodiment can by synchronized with a number of wireless sensors 01. Whenever it receives any synchronized sensor ID, it can display (when the display button is pressed) the room number and the bed number set during the synchronizing process.

The wireless display 02 in the present embodiment may also have the function of remote data and voice transmission (for carrying out voice and data exchange/transmission with the remote data terminal 03 of the system). FIG. 11 shows a remote transmitting device and a remote receiving device connected to the processor. The remote transmitting device may include a remote data transmitting device, a remote voice transmitting device and a microphone. The remote receiving device may include a remote data receiving device, a remote voice receiving device and a speaker. It may also have a set of intercom control buttons for controlling the intercom. There may also be a condition-clearing button for clearing the alarm/call condition after the sensor alarm or the call signal has been dealt with. There may be antenna and electric wires for connecting the remote transmitting device and the remote receiving device. Communication with the remote data terminal 03 can be carried out via radio frequency or electric carrier wave. In practice, they can be connected by special connecting wires.

To realize the function of address searching needed in the intercom system, there can be an address setting device for setting the address of the remote display. This allows the remote data terminal 03 to search the address of the remote display, and to effectively identify the source of all wireless data received. The address setting device can be a hardware setting device (e.g. a binary toggle switch, etc) or can employ software setting. In a software device, the address setting device can be an address setting button for setting the address code (room number).

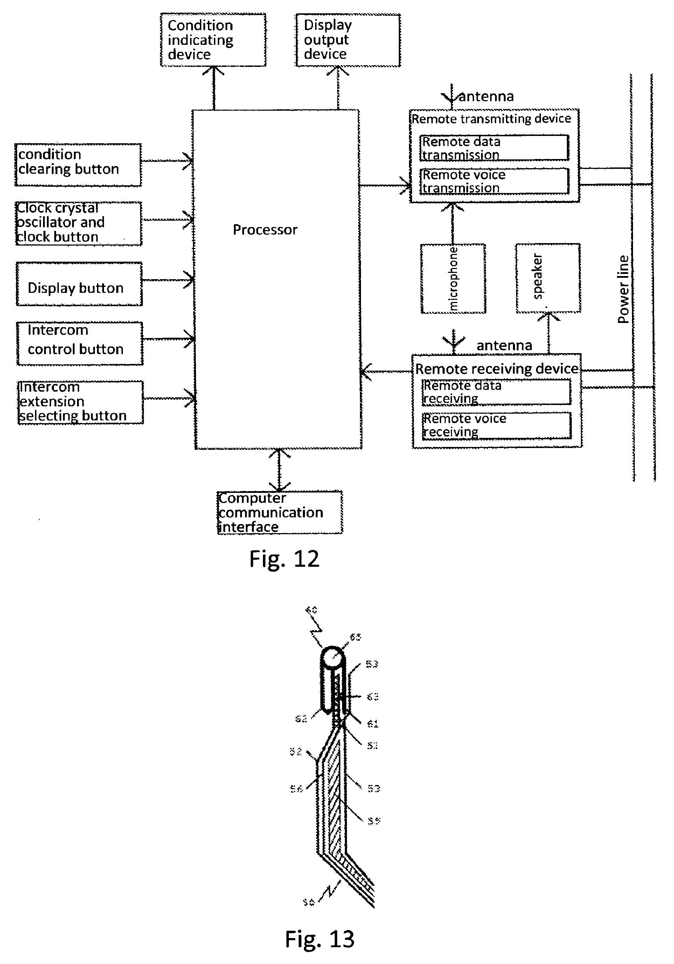

FIG. 12 is a block diagram of the remote data terminal 03, in accordance with the second aspect of the present application. It may include a remote transmitting device (including a remote data transmitting device, a remote voice transmitting device and a microphone) and a remote receiving device (including a remote data receiving device, a remote voice receiving device and a speaker) for data and voice communication with the wireless display 02. It may also include an intercom extension selecting button (select the wireless display 02 with which the intercom is carried out) and an intercom control button to realize the function of voice intercom. Same as the wireless display 02, the remote data terminal 03 of the present embodiment may also include a clock function. Hence, it may also include a clock crystal oscillator, a clock button, and a display output device, etc. The remote data terminal may further include a condition indicating device for indicating the condition of the entire monitoring alarm system, including the urine-wetness alarm condition. Same as the wireless display 02, pressing the display button can display the room number and the bed number of the sensor that generates the alarm signal when the system is in the alarm condition, and clearing the alarm condition by pressing the condition-clearing button.

The remote data terminal 03 is special in that it can further include a computer communication interface for connecting the computer and the exchanging data and information. For example, it can display on the computer screen the condition information of all sensors in the system. It can also save the condition information in a log for record purposes. It can also control condition indication and the displayed content of the wireless display 02 and the remote data terminal 03 by the computer. For example, it can clear the alarm condition. Furthermore, it can carry out the setting of time and synchronization of the wireless display 02 and the remote data terminal 03 by the computer.

In practice, the wireless sensors 01 can have many different designs to realize the cooperation of the electronic wetness-sensing diapers. FIG. 13 shows yet another embodiment of the electronic induction diaper and urine-wetness alarm of the wireless sensor 01. A side cross sectional view of the electronic induction diaper is denoted by reference numeral 50. Reference numeral 52 represents the outer layer (anti-leakage layer) of the diaper 50. Reference numeral 53 represents the inner layer (dry layer) of the diaper 50. Reference numeral 55 represents the interlayer being adhered to the outer layer and the inner layer at the waistline 51 to prevent the leakage of urine. The above-mentioned structures are the same as an ordinary diaper. The difference is that the diaper 50 in the present embodiment further includes a urine-wetness induction wires 56 located between the interlayer 55 and the outer layer 52 (in practical application, it can be located between the dry layer 53 and the interlayer 55) and extended towards the waistline region of the diaper 50.

A side view of the urine-wetness alarm of the present embodiment is denoted by reference numeral 60. The alarm may include two main components 61, 62. In use, the first component 61 can be inserted into the waistline region of the diaper 50, and can be located between the dry layer 53 and the conductive induction wires 56. This is a space reserved (e.g. without adhesive) during manufacturing. It can also be formed by tearing the inner layer 53 and the conductive induction wires apart during use. The first component may include conductive electrodes 63. The conductive electrodes 63 may be formed of conductive leaf springs or conductive rubber. When the first component 61 is inserted into the waistline of the diaper 50, the conductive electrodes 63 can be connected to the conductive induction wires 56, and transmit the urine-wetness induction signal to the second component 62 of the urine-wetness induction alarm. The second component 62 may be located outside the outer layer of the diaper 50. The second component 62 may include urine-wetness induction unit/control unit for processing the signal generated by the urine-wetness induction wires and determining whether the diaper 50 is wetted by urine or not. If the diaper 50 is wetted, an alarm signal will be transmitted through an acousto-optic alarm unit or wireless transmitting unit so as to realize the function of a urine-wetness alarm.

There may be a movable connecting component 65 between the first component 61 and the second component 62. The connecting component 65 can be a hinge and fastening device for opening the first and second components 61, 62 to facilitate the pulling out of the urine-wetness induction alarm 60 from the diaper 50, or for closing the first and second components 61, 62 so as to tightly fasten the induction wires 56 to the outer layer 52 of the diaper 50. As far as the urine-wetness induction alarm is concerned, it can achieve a fastening effect. Apart from using springs, inverted catches may be added between the first and second components 61, 62 to achieve the fastening effect.

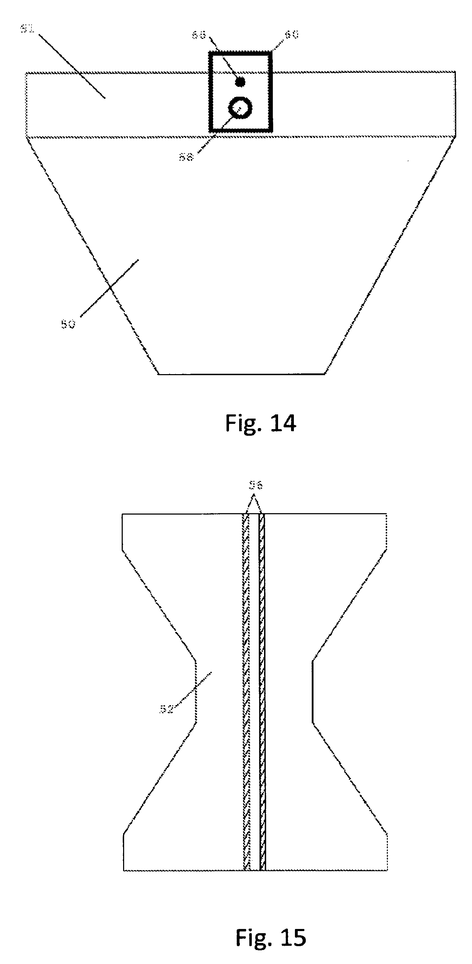

FIG. 14 is an illustrative diagram (front view) showing the structure/assembly of the electronic induction diaper and urine-wetness alarm of the wireless sensor 01 of Figure 13. Reference numeral 50 represents the main body of the diaper, and reference numeral 51 represents the waistline of the diaper 50. It can be seen that the urine-wetness induction alarm 60 can be placed at the waistline 51 of the diaper 50 normally at the front thereof. When a patient is lying on the back, the urine-wetness induction alarm 60 can be placed at the back of the diaper 50. Reference numeral 66 represents an acousto-optic indicting device provided on the urine-wetness induction alarm 60 for indicating the urine-wetness condition. If the wireless transmitting device of the alarm is not in use, the acousto-optic indicating device can be used alone as an acousto-optic alarm. This is more suitable for use by babies because babies usually do not wear pants and the acousto-optic alarm can be directly visible. When it is used by adults, the sound can be shielded off and the wireless urine-wetness alarm can be adopted. There may also be a call/trigger button 68. When the button 68 is pressed, the urine-wetness induction alarm generates an alarm signal to realize the function of emergency call of patients/elders.

The conductive induction wires of the electronic diaper that cooperate with the wireless sensor 01 may also have different designs. FIG. 15 is an illustrative diagram of an embodiment of the urine-wetness induction wires being printed on an inner edge of the outer layer of the diaper, in accordance with the second aspect of the present application. Reference numeral 52 denotes an exposed view of the outer layer of the diaper. Reference numeral 56 represents two urine-wetness induction wires printed on the inner edge of the outer layer of the diaper using conductive ink. In practical application, the induction wires 56 can be formed by directly adhering soft conductive cloth, conductive aluminum foil or other conductive material on the inner edge of the outer layer of the diaper. Normally, the length of the induction wires 56 can be the same as the length of the diaper. In use, the alarm can be placed at the front or the back at the waistline of the diaper. To save cost, only one end of the induction wires extend to the waistline and the alarm can only be placed at that one side having the induction wires.

Apart from forming the urine-wetness induction wires on the inner edge of the outer layer of the diaper using by conductive ink printing, one can also print the urine-wetness induction wires on special soft insulating band and then place/adhere the insulating band on the inner edge of the outer layer of the diaper. The advantage of this method is that the induction wires can be printed on special insulating material, which is not related to the material of the diaper, To a certain degree, this can enhance the flexibility of diaper manufacturing. In practical application, one can form the induction wires by directly adhering the soft conductive cloth, conductive aluminum foil or other conductive material on the insulating band, and then form the electronic diaper by placing/adhering the insulating band on the inner edge of the outer layer of the diaper.

It can be seen from the above that the structure of the electronic induction diapers of the embodiment disclosed in the present application are simple and easy to use. Their manufacturing process is almost the same as that of the traditional diapers. It only requires the use of an outer layer (anti-leakage layer) with urine-wetness induction wires to produce the diaper, or place a urine-wetness induction band on the inner edge of the outer layer of the diaper during the manufacturing process. It does not require special cutting or machine processing. This is beneficial in increasing the manufacturing efficiency and reducing the manufacturing cost. When the electronic induction function is not required (i.e. no need to equip with the alarm), the outer appearance and the usage of the diapers disclosed in the present application are the same as a traditional diaper. That means it can easily achieve two usages with one diaper. This is a characteristic of the technology of the present embodiment.

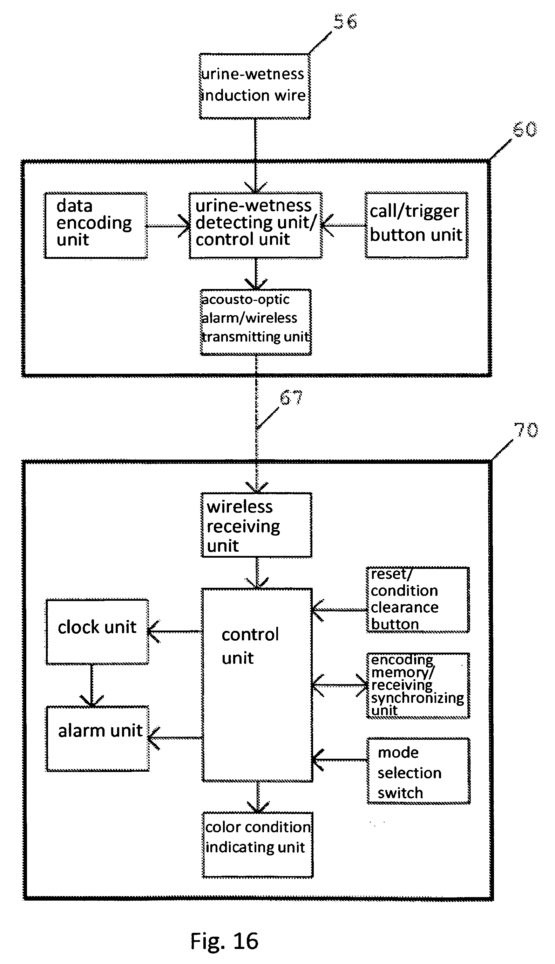

Another embodiment described below is a wireless urine-wetness alarm device formed of a wireless sensor 01 and a wireless display 02 with color condition indication, in accordance with the second aspect of the present application. FIG. 16 is a block diagram of the structure of the device. Reference numeral 56 represents a urine-wetness induction wires disposed inside the diaper. Reference numeral 60 represents a urine-wetness alarm (wireless sensor) placed at the waistline of the diaper. Reference numeral 70 represents a wireless receiver (wireless display). The urine-wetness alarm may include a urine-wetness detecting unit/monitoring unit for detecting urine-wetness. When urine-wetness condition is detected (resistance between the urine-wetness induction wires reduces), urine-wetness alarm can be carried out through acousto-optic alarm/wireless transmitting unit. A call/trigger button unit may also be connected to the urine-wetness detecting unit/monitoring unit. This can be used not only for making emergency call/summon, but can also be used for testing and transmitting alarm data encoding during data encoding "learning" process of the wireless receiver.

When the wireless transmitting unit of the urine-wetness alarm is activated, the wireless alarm signal 67 generated by the wireless sensor 60 can be wirelessly transmitted. The wireless alarm signal 67 may include special data encoding (learning code or hopping code, etc.) for the alarm in order to identify/distinguish different alarm signals. This data encoding can be provided by the data encoding unit of the alarm. The wireless alarm signal 67 can be received by the wireless receiving unit of the wireless display, and transmitted to the monitoring unit of the wireless receiver for processing. The monitoring unit can compare the data encoding in the wireless signal to the data encoding in the receiver encoding memory/receiving synchronizing unit. If they are the same or related, then it can be confirmed that the received wireless alarm signal is valid. The data encoding in the receiver can be transmitted from the designated alarm through the earlier "learning" or "synchronizing" process. Hence, the receiver can identify the designated wireless signal.

After a valid urine-wetness alarm signal is confirmed, the monitoring unit can display the urine-wetness condition by the color condition-indicating unit. Urine-wetness condition may have three display modes. In the first display mode, different colors may be used to represent different alarm signals. Hence, a wireless receiver can receive and display a number of alarm signals. This is particularly suitable for situation where a room has a number of alarms. In the second display mode, different colors may be used to represent different time of alarm. For example, the indicating unit can display green color when the alarm signal is just received. If the condition-indicating unit has not been reset after one hour, the indicating unit can automatically turn to a yellow color. If the condition-indicating unit has not been reset after two hours, the indicating unit can automatically turn to a red color until the indicating unit is reset. This function can assist a caretaker to determine the urgency of diaper changing. For example, the caretaker can change those diapers with indicating unit in red color, and then those diapers with indicating unit in yellow color and finally those diapers with indicating unit in green color. In the third display mode, a number of colors can be displayed alternately. This has a flashing light effect and can assist the caretaker to pay attention (for alternate display of a number of colors, one can adopt a single color-changing LED, or a number of LEDs of different colors). When the wetted diaper has been changed, the caretaker can reset/clear the color condition-indicating unit by using the reset/condition-clearing button on the receiver. The system can then be reset to its initial condition. In practice, one can reset the wireless receiver/display by using reset command transmitted by the wireless sensor.

A number of LEDs of different colors can be used for color display. These LEDs can be installed on the outer casing of the wireless receiver, or inside a transparent outer casing of the receiver to show a special display effect. Also, these LEDs can be used as the background light source of the built-in clock display of the wireless receiver. This can also demonstrate a special display effect.

The wireless display of the present embodiment may also include a clock unit and an alarming unit for use as a normal clock. It can also be used to display the time of the alarm, i.e. the period of time for which the urine-wetness alarm has occurred. Different functions/display modes can be carried out through a mode-selecting unit connected to the monitoring unit.

While the disposable diaper condition detecting device and monitoring system disclosed in the present application have been shown and described with particular references to a number of preferred embodiments thereof, it should be noted that various other changes or modifications may be made without departing from the scope of the appending claims.

## Claims

1. A disposable diaper condition detecting device comprises a disposable diaper (10, 50), conductive induction wires and an alarming device (20),
the disposable diaper (10, 50) comprising an outer layer (15, 52), an interlayer (16, 55) and an inner layer (17, 53);
the conductive induction wires being disposed on the outer layer (15, 52), or between the outer (15, 52) and inner layers (17, 53), for detecting urine-wetness condition of the disposable diaper (10, 50) and generating a urine-wetness condition induction signal;
the alarming device (20) being integrally formed with the conductive induction wires, or combinable with or separable from the conductive induction wires, and
the alarming device (20) being electrically coupled to the conductive induction wires when combined together for producing an alarm according to the urine-wetness condition induction signal generated by the conductive induction wires, **characterized by**
sampling means for activating the conductive induction wires to sample for the presence of urine, the sampling means including a battery-chargeable capacitor (C1) attached at the input end of the conductive induction wires, a sample input loop being triggered when a charge of the capacitor (C1) reaches a predetermined level, wetness monitoring means for determining wetness and degree of wetness, whereby a processor determines the wetness and the degree of wetness of the diaper (10, 50) according to the time triggered by the charging of the capacitor (C1).

2. A device as claimed in claim 1, **characterized in that**
the conductive induction wires are urine-wetness induction wires disposed between the outer (15, 52) and inner layers (17, 53) of the diaper (10, 50) with at least one end being extended to a waistline region (11,51) of the diaper (10, 50); and that
the alarming device (20) is a urine-wetness alarm formed by a first component (61) which is disposed at the waistline region (11, 51) in the interlayer (16, 55) between the urine-wetness induction wires and the inner layer (17, 53) of the diaper (10, 50), conductive electrodes of the alarming device (20) being in contact with the conductive induction wires, and a second component (62) which is disposed outside the outer layer (15, 52) of the diaper (10, 50); and
the urine-wetness alarm further comprises hinge and fastening devices (65) for opening the first and second components (61, 62) in order to take the urine-wetness alarm out of the diaper (10, 50), and closing the first and second components (61, 62) in order to tightly clip the urine-wetness induction wires and the outer layer (15, 52) of the diaper (10, 50).

3. A device as claimed in claim 2, **characterized in that** the device further comprises a wireless receiver (70);
the wireless receiver (70) comprising an encoding memory/receiving synchronizing unit; and
the urine-wetness alarm further comprising a data encoding unit and a wireless transmitting unit, rendering the combination of the urine-wetness alarm and the conductive induction wires to form a wireless sensor (01) for transmitting diaper condition signals or urine-wetness alarm signals.

4. A device as claimed in claim 2, **characterized in that**
the urine-wetness alarm comprises a call/trigger button unit (28, 38, 68); and
the wireless receiver (70) comprises a color condition-indicating unit; and
the wireless receiver (70) comprises a reset/condition-clearing button (25) for resetting the device, or clearing the color condition of the receiver, or receiving commands from the urine-wetness alarm in order to realize automatic condition clearance.

5. A device as claimed in claim 4, **characterized in that**
the wireless receiver (70) comprises a clock unit; and
the color condition-indicating unit comprises a color-changing LED or LEDs of different colors; and
the LED is disposed inside a transparent casing of the wireless receiver (70), or behind a display screen of a digital clock as a background light source of the display of the digital clock.

6. A device as claimed in claim 1, **characterized in that**
the device further comprises a disposable urine-wetness sensor which comprises sensing electrodes (31, 32a) and insulating gasket (30a) that are fixed outside the diaper (10, 50) when in use;
the urine-wetness alarm comprises a body device (30b) and contact electrodes (32b);
the urine-wetness sensor and the urine-wetness alarm comprise positioning devices (34a, 34b, 35a, 35b) to achieve proper combination of the urine-wetness sensor and the urine-wetness alarm.

7. A device as claimed in claim 6, **characterized in that**
the sensing electrodes (31, 32a) of the urine-wetness sensor are metal electrodes extending from one side of the insulating gasket (30a), through the gasket (30a), and to the other side of the gasket (30a);
the insulating gasket (30a) comprising a non-drying adhesive coating (33) for adhering the urine-wetness sensor onto the diaper (10, 50);
the contact electrodes (32b) of the urine-wetness alarm further comprising switch electrodes, the switch electrodes being connected and the urine-wetness alarm entering a normal operation mode when the urine-wetness sensor and the urine-wetness alarm are in a combined condition, and the switch electrodes being separated and the urine-wetness alarm entering an energy-saving, or a closed mode when the urine-wetness sensor and the urine-wetness alarm are in a separated condition.

8. A device as claimed in claim 7, **characterized in that**
conductive induction wires are disposed between the outer layer (15, 52) and the interlayer (16, 55) of the diaper (10, 50), and the outer layer (15, 52) of the diaper (10, 50) comprises a slit opening, which comprises a protective layer (89) for preventing leakage at the slit opening, and the conductive induction wire (88) is adapted to flip over the side of the slit opening and exposed outside the diaper (10, 50) so as to facilitate the installation of the urine-wetness sensor at the slit opening and the realization of electrical coupling of the sensing electrodes (31, 32a) of the urine-wetness sensor and the conductive induction wires; or
conductive induction wires (88) are disposed between the outer layer (15, 52) and the interlayer (16, 55) of the diaper (10, 50), and the outer layer (15, 52) of the diaper (10, 50) comprises a slit string for pulling open a slit opening, and the conductive induction wire (88) is adapted to flip over the side of the slit opening and exposed outside the diaper (10, 50) so as to facilitate the installation of the urine-wetness sensor at the slit opening and the realization of electrical coupling of the sensing electrodes (31, 32a) of the urine-wetness sensor and the conductive induction wires (88).

9. A device as claimed in claim 1, **characterized in that**
the diaper (10, 50) comprises a stand-alone dry space (12) accessible from the outside of the diaper (10, 50) in use;
the alarming device (60) is disposed inside the stand-alone dry space (12) to realize urine-wetness induction and alarm functions;
the conductive induction wires (56) are disposed inside the stand-alone dry space (12) on the inner edge of the outer layer (15, 52), at least a portion of the conductive induction wires (56) being extended across the dry space (12) and connected to the interlayer (16, 55) of the diaper (10, 50) so as to render electrical coupling between the stand-alone dry space and the interlayer (16, 55) of the diaper (10, 50);
the alarming device (60) is a urine-wetness alarm comprising contact electrodes (32b) so that when the urine-wetness alarm is disposed inside the stand-alone dry space (12), its contact electrodes (32b) and the interlayer (16, 55) of the diaper (10, 50) are electrically coupled; and
the urine-wetness alarm comprises a resilient clip (22) for holding the urine-wetness alarm inside the stand-alone dry space (12) and making the electrical coupling between the contact electrodes (32b) of the urine-wetness alarm and the interlayer (16, 55) of the diaper (10, 50) more reliable.

10. A disposable diaper condition monitoring system, **characterized in that** the system comprises a disposable diaper condition detecting device as claimed in any one of the preceding claims and a disposable diaper condition displaying device, the disposable diaper condition displaying device being adapted to display diaper condition signals according to urine-wetness condition signals or urine-wetness alarm signals transmitted from the detecting device;
the disposable diaper condition detecting device further comprises a wireless sensor, the wireless sensor comprising a sensing input device, transmittable sensor ID, and a wireless transmitting device, the transmittable sensor ID is transmitted via the wireless transmitting device when the sensor enters a triggered condition; and
the disposable diaper condition displaying device comprises a wireless display (02), the wireless display (02) comprising a wireless receiving device, a data processing device, and a displaying device, whereby after the display receives the transmittable sensor ID transmitted from the sensor, the data processing device determines its relationship with the display and, if related, displays on the displaying device the signal related to the sensor.

11. A system as claimed in claim 10, **characterized in that** the wireless display (02) further comprises a sensor ID synchronizing device and sensor ID synchronizing list, rendering the establishment of a corresponding display relationship between the wireless display (02) and the wireless sensor (01), and that the display relationship is set by a user.

12. A system as claimed in claim 11, **characterized in that** the system further comprises:
a remote data terminal (03) comprising a remote data receiving device, a display output device or computer interface device for receiving and displaying data signals from the wireless display (02); and
the wireless display (02) comprising an address setting device and a remote data transmitting device for realizing data transmission with the remote data terminal (03).

13. A system as claimed in claim 12, **characterized in that** the wireless display (02) and the remote data terminal (03) comprise a remote data transmitting device, a remote data receiving device, a remote voice transmitting device, a remote voice receiving device and an intercom control device for realizing data and voice communication between the wireless display (02) and the remote data terminal (03).

## Patentansprüche

1. Wegwerfwindelzustandsdetektor, der Folgendes umfasst:
eine Wegwerfwindel (10, 50), leitfähige Induktionsleitungen und eine Alarmierungsvorrichtung (20), wobei
die Wegwerfwindel (10, 50) eine Außenschicht (15, 52), eine Zwischenschicht (16, 55) und eine Innenschicht (17, 53) umfasst;
die leitfähigen Induktionsleitungen auf der Außenschicht (15, 52) oder zwischen den Außen- (15, 52) und Innenschichten (17, 53) angeordnet sind, um den Urinnässezustand der Wegwerfwindel (10,50) zu erkennen und um ein Induktionssignal für den Urinnässezustand zu erzeugen;
die Alarmierungsvorrichtung (20) integral mit den leitfähigen Induktionsleitungen ausgebildet ist oder mit den leitfähigen Induktionsleitungen kombinierbar oder von diesen abtrennbar ist, und
die Alarmierungsvorrichtung (20), wenn sie kombiniert angeordnet ist, mit den leitfähigen Induktionsleitungen elektrisch gekoppelt ist, um einen Alarm entsprechend dem von den leitfähigen Induktionsleitungen erzeugten Induktionssignal für den Urinnässezustand zu erzeugen, **gekennzeichnet durch**
Probenahmemittel zum Aktivieren der leitfähigen Induktionsleitungen, um eine Stichprobe auf Vorhandensein von Urin durchzuführen, wobei die Probenahmemittel Folgendes umfassen: einen batterieaufladbaren Kondensator (C1), der am Eingangsende der leitfähigen Induktionsleitungen angebracht ist, eine Probeeingangsschleife, die aktiviert wird, wenn eine Ladung des Kondensators (C1) eine festgelegte Höhe erreicht, Nässeüberwachungsmittel zum Ermitteln der Nässe und des Nässegrads, wobei ein Prozessor die Nässe und den Nässegrad der Windel (10, 50) entsprechend der von der Ladung des Kondensators (C1) aktivierten Zeit ermittelt.

2. Vorrichtung nach Anspruch 1, **dadurch gekennzeichnet, dass**
die leitfähigen Induktionsleitungen Urinnässe-Induktionsleitungen sind, die zwischen den Außen- (15, 52) und den Innenschichten (17, 53) der Windel (10, 50) angeordnet sind, wobei sich mindestens ein Ende bis zu einem Taillenbereich (11, 51) der Windel (10, 50) erstreckt; und dadurch, dass
die Alarmierungsvorrichtung (20) eine Urinnässe-Alarmeinheit ist, die aus einer ersten Komponente (61), die in dem Taillenbereich (11, 51) in der Zwischenschicht (16, 55) zwischen den Urinnässe-Induktionsleitungen und der Innenschicht (17, 53) der Windel (10, 50) angeordnet ist, leitfähigen Elektroden der Alarmierungsvorrichtung (20), die mit den leitfähigen Induktionsleitungen in Kontakt sind, und einer außerhalb der Außenschicht (15, 52) der Windel (10, 50) angeordneten zweiten Komponente (62) gebildet wird; und dadurch, dass
die Urinnässe-Alarmeinheit weiterhin Scharnier- und Befestigungsvorrichtungen (65) zum Öffnen der ersten und zweiten Komponenten (61, 62) umfasst, um die Urinnässe-Alarmeinheit aus der Windel (10, 50) herauszunehmen, und zum Schließen der ersten und zweiten Komponenten (61, 62), um die Urinnässe- Induktionsleitungen und die Außenschicht (15, 52) der Windel (10, 50) fest zusammenzuhalten.

3. Vorrichtung nach Anspruch 2, **dadurch gekennzeichnet, dass** die Vorrichtung weiterhin einen Drahtlosempfänger (70) umfasst;
wobei der Drahtlosempfänger (70) einen Codierspeicher/eine synchronisierende Empfangseinheit umfasst; und
die Urinnässe-Alarmeinheit weiterhin eine Datencodiereinheit und eine kabellose Übertragungseinheit umfasst, die die Kombination aus Urinnässe-Alarmeinheit und den leitfähigen Induktionsleitungen ergibt, um einen kabellosen Sensor (01) zum Übertragen der Windelzustandssignale oder Urinnässe-Alarmsignale zu bilden.

4. Vorrichtung nach Anspruch 2, **dadurch gekennzeichnet, dass**
die Urinnässe-Alarmeinheit eine Ruf-/Aktivierknopfeinheit (28, 38, 68) umfasst; und
der Drahtlosempfänger (70) eine Farbzustandsanzeigeeinheit umfasst; und
der Drahtlosempfänger (70) eine Reset-/ Zustandslöschtaste (25) zum Rücksetzen der Vorrichtung oder zum Löschen des Farbzustands des Empfängers, oder zum Empfangen von Befehlen von der Urinnässe-Alarmeinheit bezüglich des Durchführens einer automatischen Zustandslöschung umfasst.

5. Vorrichtung nach Anspruch 4, **dadurch gekennzeichnet, dass**
der Drahtlosempfänger (70) ein Uhrmodul umfasst; und
die Farbzustandsanzeigeeinheit eine farbwechselnde LED oder LEDs mit verschiedenen Farben umfasst; und
die LED in einem transparenten Gehäuse des Drahtlosempfängers (70) oder hinter einem Bildschirm einer Digitaluhr als eine Hintergrundlichtquelle des Displays der Digitaluhr angeordnet ist.

6. Vorrichtung nach Anspruch 1, **dadurch gekennzeichnet, dass**
die Vorrichtung weiterhin einen Einweg-Urinnässesensor umfasst, der Messelektroden (31, 32a) und eine Isolierdichtung (30a) umfasst, die bei Gebrauch außerhalb der Windel (10, 50) angebracht sind;
die Urinnässe-Alarmeinheit eine Körpervorrichtung (30b) und Kontaktelektroden (32b) umfasst;
der Urinnässesensor und die Urinnässe-Alarmeinheit Positioniervorrichtungen (34a, 34b, 35a, 35b) umfassen, um eine angemessene Kombination aus Urinnässesensor und Urinnässe-Alarmeinheit zu erreichen.

7. Vorrichtung nach Anspruch 6, **dadurch gekennzeichnet, dass**
die Messelektroden (31, 32a) des Urinnässesensors Metallelektroden sind, die sich von einer Seite der Isolierdichtung (30a) durch die Dichtung (30a) und zur anderen Seite der Dichtung (30a) erstrecken;
die Isolierdichtung (30a) eine nicht-trocknende Klebebeschichtung (33) zum Ankleben des Urinnässesensors an der Windel (10, 50) umfasst;
die Kontaktelektroden (32b) der Urinnässe-Alarmeinheit weiterhin Schaltelektroden umfassen, wobei die Schaltelektroden verbunden sind und die Urinnässe-Alarmeinheit in einen Normalbetrieb schaltet, wenn sich der Urinnässesensor und die Urinnässe-Alarmeinheit in einem kombinierten Zustand befinden, und wobei die Schaltelektroden getrennt sind und die Urinnässe-Alarmeinheit in einen energiesparenden oder geschlossenen Betrieb schaltet, wenn sich der Urinnässesensor und die Urinnässe-Alarmeinheit in einem getrenntem Zustand befinden.

8. Vorrichtung nach Anspruch 7, **dadurch gekennzeichnet, dass**
leitfähige Induktionsleitungen zwischen der Außenschicht (15, 52) und der Zwischenschicht (16, 55) der Windel (10, 50) angeordnet sind, und die Außenschicht (15, 52) der Windel (10, 50) eine Schlitzöffnung umfasst, die eine Schutzschicht (89) zum Vermeiden von Auslaufen an der Schlitzöffnung umfasst, und die leitfähige Induktionsleitung (88) dazu geeignet ist, über die Seite der Schlitzöffnung und die freiliegende Außenseite der Windel (10, 50) geschlagen zu werden, um die Installation des Urinnässesensors an der Schlitzöffnung und die Realisierung einer elektrischen Kopplung der Messelektroden (31, 32a) des Urinnässesensors und der leitfähigen Induktionsleitungen zu vereinfachen; oder
die leitfähigen Induktionsleitungen (88) zwischen der Außenschicht (15, 52) und der Zwischenschicht (16, 55) der Windel (10, 50)angeordnet sind, und die Außenschicht (15, 52) der Windel (10, 50) einen Schlitzfaden zum Öffnen einer Schlitzöffnung umfasst, und die leitfähige Induktionsleitung (88) dazu geeignet ist, über die Seite der Schlitzöffnung und die freiliegende Außenseite der Windel (10, 50) geschlagen zu werden, um die Installation des Urinnässesensors an der Schlitzöffnung und die Realisierung einer elektrischen Kopplung der Messelektroden (31, 32a) des Urinnässesensors und der leitfähigen Induktionsleitungen (88) zu vereinfachen.

9. Vorrichtung nach Anspruch 1, **dadurch gekennzeichnet, dass**
die Windel (10, 50) einen unabhängigen Trockenbereich (12) umfasst, der von außerhalb der in Gebrauch befindlichen Windel (10, 50) zugänglich ist;
die Alarmierungsvorrichtung (60) innerhalb des unabhängigen Trockenbereichs (12)angeordnet ist, um Urinnässe-Induktion und Alarmfunktionen zu realisieren;
die leitfähigen Induktionsleitungen (56) innerhalb des unabhängigen Trockenbereichs (12) an der Innenkante der Außenschicht (15, 52) angeordnet sind, wobei sich mindestens ein Teil der leitfähigen Induktionsleitungen (56) durch den Trockenbereich (12) erstreckt und mit der Zwischenschicht (16, 55) der Windel (10, 50) verbunden ist, um eine elektrische Kopplung zwischen dem unabhängigen Trockenbereich und der Zwischenschicht (16, 55) der Windel (10, 50) herzustellen;
die Alarmierungsvorrichtung (60) eine Urinnässe-Alarmeinheit ist, der Kontaktelektroden (32b) umfasst, so dass bei Anordnung der Urinnässe-Alarmeinheit innerhalb des unabhängigen Trockenbereichs (12) ihre Kontaktelektroden (32b) und die Zwischenschicht (16, 55) der Windel (10, 50) elektrisch gekoppelt sind; und
die Urinnässe-Alarmeinheit einen elastischen Clip (22) umfasst, der die Urinnässe-Alarmeinheit innerhalb des unabhängigen Trockenbereichs (12) hält und die elektrische Kopplung zwischen den Kontaktelektroden (32b) der Urinnässe-Alarmeinheit und der Zwischenschicht (16, 55) der Windel (10, 50) zuverlässiger macht.

10. Einwegwindelzustandsüberwachungssystem, **dadurch gekennzeichnet, dass** das das System einen Einwegwindelzustandsdetektor nach einem der vorhergehenden Ansprüche umfasst, und eine Einwegwindelzustandsanzeigevorrichtung, wobei die Einwegwindelzustandsanzeigevorrichtung geeignet ist, Windelzustandssignale entsprechend den vom Detektor übertragenen Urinnässe-Zustandssignalen oder Urinnässe-Alarmsignalen anzuzeigen;
der Einwegwindelzustandsdetektor weiterhin einen drahtlosen Sensor umfasst, wobei der drahtlose Sensor eine abtastende Eingabevorrichtung, übertragbare Sensor-ID und eine drahtlose Übertragungsvorrichtung umfasst, wobei die übertragbare Sensor-ID über die drahtlose Übertragungsvorrichtung übertragen wird, wenn der Sensor in einen aktivierten Zustand übergeht; und
die Einwegwindelzustandsanzeigevorrichtung eine drahtlose Anzeige (02) umfasst, wobei die drahtlose Anzeige (02) eine drahtlose Empfangsvorrichtung, eine Datenverarbeitungsvorrichtung und eine Anzeigevorrichtung umfasst, wobei die Datenverarbeitungsvorrichtung, nachdem die Anzeige die vom Sensor gesendete übertragbare Sensor-ID empfangen hat, ihre Verknüpfung mit der Anzeige ermittelt, und, falls eine Verknüpfung besteht, das dem Sensor zugeordnete Signal auf der Anzeigevorrichtung anzeigt.

11. System nach Anspruch 10, **dadurch gekennzeichnet, dass** die drahtlose Anzeige (02) weiterhin eine Sensor-ID-Synchronisiervorrichtung und eine Sensor-ID-Synchronisierliste umfasst, die die Einrichtung einer entsprechenden Anzeigeverknüpfung zwischen der drahtlosen Anzeige (02) und dem drahtlosen Sensor (01) durchführen, und dass die Anzeigeverknüpfung vom Nutzer eingestellt wird.

12. System nach Anspruch 11, **dadurch gekennzeichnet, dass** das System weiterhin Folgendes umfasst:
ein externes Datenendgerät (03), das eine externe Datenempfangsvorrichtung, eine Anzeigeausgabevorrichtung oder Computerschnittstellenvorrichtung zum Empfangen und Anzeigen von Datensignalen von der drahtlosen Anzeige (02) umfasst; und
die drahtlose Anzeige (02), die eine Adresseneinstellvorrichtung und eine externe Datenübertragungsvorrichtung zum Realisieren der Datenübertragung mit dem externen Datenendgerät (03) umfasst.

13. System nach Anspruch 12, **dadurch gekennzeichnet, dass**
die drahtlose Anzeige (02) und das externe Datenendgerät (03) eine externe Datenübertragungsvorrichtung, eine externe Datenempfangsvorrichtung, eine externe Sprachübertragungsvorrichtung, eine externe Sprachempfangsvorrichtung und eine Intercom-Steuervorrichtung zum Realisieren einer Daten- und Sprachkommunikation zwischen der drahtlosen Anzeige (02) und dem externen Datenendgerät (03) umfassen.

## Revendications

1. Dispositif de détection d'état de couche jetable qui comprend une couche jetable (10, 50), des fils à induction conducteurs et un dispositif d'alarme (20),
la couche jetable (10, 50) comprenant une couche externe (15, 52), une couche intermédiaire (16, 55) et une couche interne (17, 53) ;
les fils à induction conducteurs étant disposés sur la couche externe (15, 52), ou entre les couches externe (15, 52) et interne (17, 53), pour détecter un état d'humidité due à de l'urine de la couche jetable (10, 50) et générer un signal d'induction d'état d'humidité due à de l'urine ;
le dispositif d'alarme (20) étant formé de manière intégré avec les fils à induction conducteurs, ou apte à être combiné avec ou apte à être séparé des fils à induction conducteurs, et
le dispositif d'alarme (20) étant électriquement couplé aux fils à induction conducteurs lorsqu'ils sont combinés ensemble pour produire une alarme selon le signal d'induction d'état d'humidité due à de l'urine généré par les fils à induction conducteurs, **caractérisé par**
des moyens d'échantillonnage pour activer les fils à induction conducteurs pour échantillonner la présence d'urine, les moyens d'échantillonnage comprenant un condensateur chargeable par batterie (C1) fixé à l'extrémité d'entrée des fils à induction conducteurs, une boucle d'entrée d'échantillon étant déclenchée lorsqu'une charge du condensateur (C1) atteint un niveau prédéterminé, des moyens de surveillance d'humidité pour déterminer l'humidité et le degré d'humidité, ce par quoi un processeur détermine l'humidité et le degré d'humidité de la couche (10, 50) selon le temps déclenché par la charge du condensateur (C1).

2. Dispositif selon la revendication 1, **caractérisé par le fait que** :
les fils à induction conducteurs sont des fils à induction d'humidité due à de l'urine disposés entre les couches externe (15, 52) et interne (17, 53) de la couche (10, 50) avec au moins une extrémité qui s'étend jusqu'à une région de taille (11, 51) de la couche (10, 50) ; et **par le fait que** :
le dispositif d'alarme (20) est une alarme d'humidité due à de l'urine, formée par un premier composant (6) qui est disposé à la région de taille (11, 51) dans la couche intermédiaire (16, 55) entre les fils à induction d'humidité due à de l'urine et la couche interne (17, 53) de la couche (10, 50), des électrodes conductrices du dispositif d'alarme (20) étant en contact avec les fils à induction conducteurs, et par un second composant (62) qui est disposé à l'extérieur de la couche externe (15, 52) de la couche (10, 50) ; et
l'alarme d'humidité due à de l'urine comprend en outre des dispositifs de charnière et de fixation (65) pour ouvrir les premier et second composants (61, 62) afin de sortir l'alarme d'humidité due à de l'urine hors de la couche (10, 50), et fermer les premier et second composants (61, 62) afin de pincer fermement les fils à induction d'humidité due à de l'urine et la couche externe (15, 52) de la couche (10, 50).

3. Dispositif selon la revendication 2, **caractérisé par le fait que** le dispositif comprend en outre un récepteur sans fil (70) ;
le récepteur sans fil (70) comprenant une unité de mémoire de codage/synchronisation de réception ; et
l'alarme d'humidité due à de l'urine comprenant en outre une unité de codage de données et une unité d'émission sans fil, restituant la combinaison de l'alarme d'humidité due à de l'urine et des fils à induction conducteurs pour former un capteur sans fil (01) pour émettre des signaux d'état de couche ou des signaux d'alarme d'humidité due à de l'urine.

4. Dispositif selon la revendication 2, **caractérisé par le fait que** :
l'alarme d'humidité due à de l'urine comprend une unité de bouton d'appel/déclenchement (28, 38, 68) ; et
le récepteur sans fil (70) comprend une unité d'indication d'état à couleur ; et
le récepteur sans fil (70) comprend un bouton de réinitialisation/effacement d'état (25) pour réinitialiser le dispositif ou effacer l'état de couleur du récepteur, ou recevoir des instructions provenant de l'alarme d'humidité due à de l'urine afin de réaliser un effacement d'état automatique.

5. Dispositif selon la revendication 4, **caractérisé par le fait que** :
le récepteur sans fil (70) comprend une unité d'horloge ; et
l'unité d'indication d'état à couleur comprend une DEL à changement de couleur ou des DEL de différentes couleurs ; et
la DEL est disposée à l'interne d'un boîtier transparent du récepteur sans fil (70), ou derrière un écran de dispositif d'affichage d'une horloge numérique en tant que source lumineuse d'arrière-plan du dispositif d'affichage de l'horloge numérique.

6. Dispositif selon la revendication 1, **caractérisé par le fait que** :
le dispositif comprend en outre un capteur d'humidité due à de l'urine jetable qui comprend des électrodes de détection (31, 32a) et un joint isolant (30a) qui sont fixés à l'extérieur de la couche (10, 50) en utilisation ;
l'alarme d'humidité due à de l'urine comprend un dispositif corps (30b) et des électrodes de contact (32b) ;
le capteur d'humidité due à de l'urine et l'alarme d'humidité due à de l'urine comprennent des dispositifs de positionnement (34a, 34b, 35a, 35b) pour obtenir une combinaison correcte du capteur d'humidité due à de l'urine et de l'alarme d'humidité due à de l'urine.

7. Dispositif selon la revendication 6, **caractérisé par le fait que** :
les électrodes de détection (31, 32a) du capteur d'humidité due à de l'urine sont des électrodes métalliques s'étendant à partir d'un côté du joint isolant (30a), à travers le joint (30a) et jusqu'à l'autre côté du joint (30a) ;
le joint isolant (30a) comprenant un revêtement adhésif non séchant (33) pour faire adhérer sur la couche (10, 50) le capteur d'humidité due à de l'urine ;
les électrodes de contact (32b) de l'alarme d'humidité due à de l'urine comprenant en outre des électrodes de commutation, les électrodes de commutation étant reliées et l'alarme d'humidité due à de l'urine entrant dans un mode de fonctionnement normal lorsque le capteur d'humidité due à de l'urine et l'alarme d'humidité due à de l'urine sont dans un état combiné, et les électrodes de commutation étant séparées et l'alarme d'humidité due à de l'urine entrant dans un mode d'économie d'énergie ou un mode fermé lorsque le capteur d'humidité due à de l'urine et l'alarme d'humidité due à de l'urine sont dans un état séparé.

8. Dispositif selon la revendication 7, **caractérisé par le fait que** :
des fils à induction conducteurs sont disposés entre la couche externe (15, 52) et la couche intermédiaire (16, 55) de la couche (10, 50), et la couche externe (15, 52) de la couche (10, 50) comprend une ouverture en fente, qui comprend une couche protectrice (89) pour empêcher une fuite au niveau de l'ouverture en fente, et le fil à induction conducteur (88) est agencé pour retourner le côté de l'ouverture en fente et exposé à l'extérieur de la couche (10, 50) de façon à faciliter l'installation du capteur d'humidité due à de l'urine au niveau de l'ouverture en fente et la réalisation d'un couplage électrique des électrodes de détection (31, 32a) du capteur d'humidité due à de l'urine et des fils à induction conducteurs ; ou
des fils à induction conducteurs (88) sont disposés entre la couche externe (15, 52) et la couche intermédiaire (16, 55) de la couche (10, 50), et la couche externe (15, 52) de la couche (10, 50) comprend une chaîne de fente pour ouvrir par traction une ouverture en fente, et le fil à induction conducteur (88) est agencé pour retourner le côté de l'ouverture en fente et exposé à l'extérieur de la couche (10, 50) de façon à faciliter l'installation du capteur d'humidité due à de l'urine au niveau de l'ouverture en fente et la réalisation d'un couplage électrique des électrodes de détection (31, 32a) du capteur d'humidité due à de l'urine et des fils à induction conducteurs (88).

9. Dispositif selon la revendication 1, **caractérisé par le fait que** :
la couche (10, 50) comprend un espace sec autonome (12) accessible depuis l'extérieur de la couche (10, 50) en utilisation ;
le dispositif d'alarme (60) est disposé à l'intérieur de l'espace sec autonome (12) pour réaliser les fonctions d'alarme et d'induction d'humidité due à de l'urine ;
les fils à induction conducteurs (56) sont disposés à l'intérieur de l'espace sec autonome (12) sur le bord interne de la couche externe (15, 52), au moins une partie des fils à induction conducteurs (56) s'étendant en travers de l'espace sec (12) et étant reliée à la couche intermédiaire (16, 55) de la couche (10, 50) de façon à restituer un couplage électrique entre l'espace sec autonome et la couche intermédiaire (16, 55) de la couche (10, 50) ;
le dispositif d'alarme (60) est une alarme d'humidité due à de l'urine comprenant des électrodes de contact (32b) telles que lorsque l'alarme d'humidité due à de l'urine est disposée à l'intérieur de l'espace sec autonome (12), ses électrodes de contact (32b) et la couche intermédiaire (16, 55) de la couche (10, 50) sont électriquement couplées ; et
l'alarme d'humidité due à de l'urine comprend une attache élastique (22) pour maintenir l'alarme d'humidité due à de l'urine à l'intérieur de l'espace sec autonome (12) et rendre plus fiable le couplage électrique entre les électrodes de contact (32b) de l'alarme d'humidité due à de l'urine et la couche intermédiaire (16, 55) de la couche (10, 50).

10. Système de surveillance d'état de couche jetable, **caractérisé par le fait que** le système comprend un dispositif de détection d'état de couche selon l'une quelconque des revendications précédentes et un dispositif d'affichage d'état de couche jetable, le dispositif d'affichage d'état de couche jetable étant agencé pour afficher des signaux d'état de couche selon des signaux d'état d'humidité due à de l'urine ou des signaux d'alarme d'humidité due à de l'urine émis par le dispositif de détection ;
le dispositif de détection d'état de couche jetable comprend en outre un capteur sans fil, le capteur sans fil comprenant un dispositif d'entrée de détection, un identificateur de capteur apte à être émis et un dispositif d'émission sans fil, l'identificateur de capteur apte à être émis est émis par l'intermédiaire du dispositif d'émission sans fil lorsque le capteur entre dans un état déclenché ; et
le dispositif d'affichage d'état de couche jetable comprend un dispositif d'affichage sans fil (02), le dispositif d'affichage sans fil (02) comprenant un dispositif de réception sans fil, un dispositif de traitement de données et un dispositif d'affichage, ce par quoi, après que le dispositif d'affichage a reçu l'identificateur de capteur apte à être émis, émis par le capteur, le dispositif de traitement de données détermine sa relation avec le dispositif d'affichage et, s'il est associé, affiche sur le dispositif d'affichage le signal associé au capteur.

11. Système selon la revendication 10, **caractérisé par le fait que** le dispositif d'affichage sans fil (02) comprend en outre un dispositif de synchronisation d'identificateur de capteur et une liste de synchronisation d'identificateur de capteur, restituant l'établissement d'une relation d'affichage correspondante entre le dispositif d'affichage sans fil (02) et le capteur sans fil (01), et **par le fait que** la relation d'affichage est réglée par un utilisateur.

12. Système selon la revendication 11, **caractérisé par le fait que** le système comprend en outre :
un terminal de données à distance (3) comprenant un dispositif de réception de données à distance, un dispositif de sortie d'affichage ou un dispositif d'interface d'ordinateur pour recevoir et afficher des signaux de données provenant du dispositif d'affichage sans fil (02) ; et
le dispositif d'affichage sans fil (02) comprenant un dispositif de réglage d'adresse et un dispositif d'émission de données à distance pour réaliser une transmission de données avec le terminal de données à distance (03).

13. Système selon la revendication 12, **caractérisé par le fait que** le dispositif d'affichage sans fil (02) et le terminal de données à distance (03) comprennent un dispositif d'émission de données à distance, un dispositif de réception de données à distance, un dispositif d'émission vocale à distance, un dispositif de réception vocale à distance et un dispositif de commande d'intercommunication pour réaliser une communication de données et vocale entre le dispositif d'affichage sans fil (02) et le terminal de données à distance (03).
